(19) Europäisches Patentamt
European Patent Office
Office européen des brevets

(11) EP 0 821 945 B1

(12) EUROPÄISCHE PATENTSCHRIFT

(45) Veröffentlichungstag und Bekanntmachung des Hinweises auf die Patenterteilung:
**05.03.2003 Patentblatt 2003/10**

(51) Int Cl.[7]: **A61K 7/42**, A61K 7/50, A61K 7/00

(21) Anmeldenummer: **97112201.5**

(22) Anmeldetag: **17.07.1997**

(54) **Schaumförmige Lichtzubereitungen mit einem Gehalt an wasserlöslichen Lichtschutzsubstanzen und grenzflächenaktiven Substanzen**

Photoprotective composition in foam form containing water soluble ultraviolet filters and surface active agents

Composition de protection solaire sous forme de mousse, contenant des filtres ultraviolette et des tensioactifs

(84) Benannte Vertragsstaaten:
**AT BE CH DE ES FR GB IT LI NL SE**

(30) Priorität: **02.08.1996 DE 19631221**

(43) Veröffentlichungstag der Anmeldung:
**04.02.1998 Patentblatt 1998/06**

(73) Patentinhaber: **Beiersdorf Aktiengesellschaft**
**20245 Hamburg (DE)**

(72) Erfinder:
- **Gers-Barlag, Heinrich, Dr.**
  **25495 Kummerfeld (DE)**
- **Müller, Anja**
  **20253 Hamburg (DE)**
- **Maurer, Peter**
  **22880 Wedel (DE)**

(56) Entgegenhaltungen:
**WO-A-96/14053**
**WO-A-96/22073**
**US-A- 5 306 486**
**US-A- 5 322 683**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische und dermatologische Lichtschutzzubereitungen, insbesondere hautpflegende kosmetische und dermatologische Lichtschutzzubereitungen. In einer besonderen Ausführungsform betrifft die vorliegende Erfindung auch nachschäumende kosmetische und dermatologische Lichtschutzzubereitungen.
Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.
Als ein Maximum der Erythemwirksamkeit des Sonnenlichtes wird der engere Bereich um 308 nm angegeben.
Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.
Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.
Die UV-Strahlung kann aber auch zu photochemischen Reaktionen führen, wobei dann die photochemischen Reaktionsprodukte in den Hautmetabolismus eingreifen.
Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.
Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.
Die 2-Phenylbenzimidazol-5-sulfonsäure bzw. ihre Salze, insbesondere das Natrium-, das Kalium und das TEA-Salz, beispielsweise erhältlich unter der Bezeichnung Eusolex® 232 der Merck AG, welches sich durch folgende Strukturformel auszeichnet:

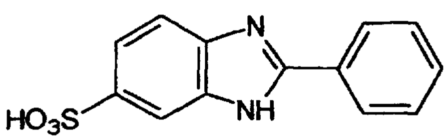

ist eine an sich vorteilhafte, in Wasser lösliche UV-Fittersubstanz.
Eine weitere bekannte und vorteilhafte, allerdings wasserunlösliche, Lichtschutzfiltersubstanz ist das 4-(tert. -Butyl)-4'-methoxydibenzoylmethan, welches sich durch die Struktur

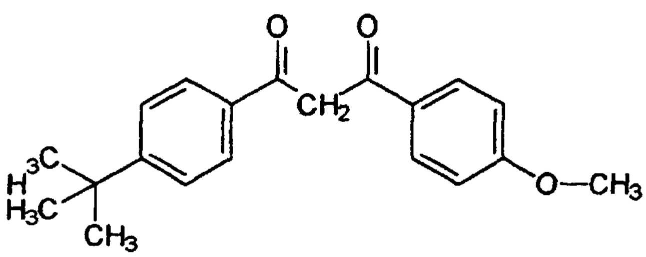

auszeichnet, und von Givaudan unter der Marke Parsol® 1789 verkauft wird
Der Hauptnachteil dieser Substanz ist eine gewisse Instabilität gegenüber UV-Strahlung, so daß es zweckmäßig ist, Zubereitungen mit einem Gehalt an dieser Substanz auch gewisse UV-Stabilisatoren einzuverleiben.

Ein weiterer vorteilhafter UVB-Filter ist der 4,4',4''-(1,3,5-Triazin-2,4,6-triyltriimino)-tris-benzoësäure-tris (2-ethylhexylester), synonym: 2,4,6-Tris-[anilino-(p-carbo-2'-ethyl-1'-hexyloxy)]-1,3,5-triazin.

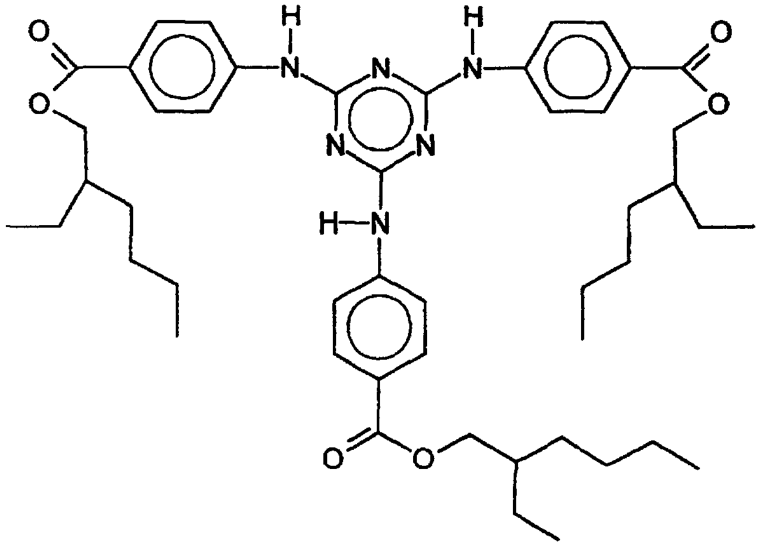

Diese UVB-Filtersubstanz wird von der BASF Aktiengesellschaft unter der Warenbezeichnung UVINUL® T 150 vertrieben und zeichnet sich durch gute UV-Absorptionseigenschaften aus.

Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz.

Eine weitere vorteilhafte Lichtschutzfiltersubstanz ist der 4-Methylbenzylidencampher, welcher sich durch die Struktur

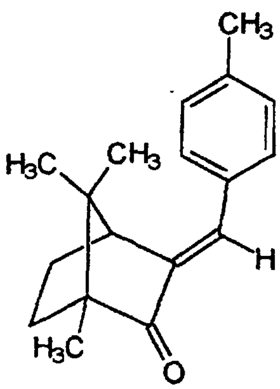

auszeichnet und von Merck unter der Marke Eusolex® 6300 verkauft wird. Diese Substanzen zeichnen sich an sich durch gute UV-Filtereigenschaften aus. Gerade in Kombination miteinander oder anderen als Festkörper vorliegenden Substanzen ist ihre Einsatzkonzentration jedoch begrenzt.

Eine weiterere vorteilhafte Lichtschutzfiltersubstanz ist das 2-Ethylhexyl-p-methoxycinnamat, welches von Givaudan unter der Bezeichnung Parsol® MCX erhältlich ist und sich durch folgende Struktur auszeichnet:

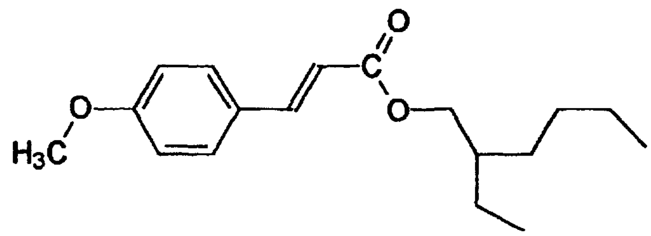

Noch eine weiterere vorteilhafte Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

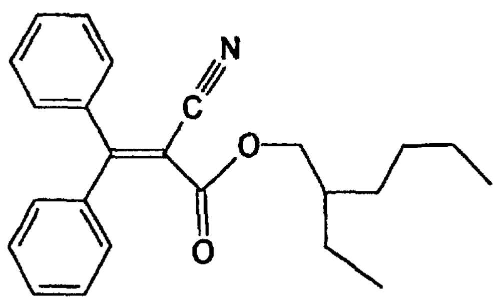

Insbesondere wenn mehrere der unter Normalbedingungen kristallin vorliegenden Lichtschutzsubstanzen vorliegen, beispielsweise gewählt aus der Gruppe 4,4',4"-(1,3,5-Triazin-2,4,6-triyltriimino)-tris-benzoesäure-tris (2-ethylhexylester), 4-Methyl-benzylidencampher, 4-(tert.-Butyl)-4-methoxydibenzoylmethan und insbesondere, wenn zusätzlich Titandioxid als unlösliche Komponente vorliegt, sind gemäß den Lehren des Standes der Technik nur jeweils geringe Einsatzkonzentrationen und damit niedrige Lichtschutzfaktoren möglich, es sei denn, der Anteil der Ölphase würde überproportional erhöht, was aber ebenfalls Nachteile hätte.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Die anorganischen Pigmente zeichnen sich an sich durch gute Lichtschutzwirkung aus. Sie haben jedoch den Nachteil, daß es schwierig ist, sie in befriedigender Weise solchen Formulierungen einzuverleiben. Nur wenn die Partikel in der endgültigen Formulierung sehr klein sind, werden sie nach dem Auftragen auf die Haut nicht als störendes "Weißeln" (Bildung von weißen Flecken auf der Haut) beobachtet. Üblicherweise liegen die Partikelgrößen solcher Pigmente im Bereich unter 100 nm. In einer herkömmlichen Emulsion neigen die Partikel mehr oder weniger stark zur Zusammenlagerung zu Agglomeraten, welche bereits unter dem Lichtmikroskop zu erkennen sind. Solche Agglomeration ist ferner nicht mit dem Herstellungsprozeß einer entsprechenden Zubereitung abgeschlossen, sondern setzt sich während der Lagerung fort. Das "Weißeln" kann sich daher über einen längeren Zeitraum noch verstärken. Auch kann das Ausölen oder gar Brechen einer Emulsion mittel- oder langfristig Folge einer derartigen Agglomeration sein.

Ein weiterer Nachteil des Einsatzes anorganischer Pigmente in kosmetischen Formulierungen ist, daß solche Pigmente in den weitaus meisten Fällen zu starker Hauttrockenheit führen.

Dennoch bestand der Nachteil des Standes der Technik, daß in der Regel entweder nur vergleichsweise niedrige Lichtschutzfaktoren erreicht werden konnten, oder daß die Lichtschutzfilter nicht die genügende UV-Stabilität aufwiesen oder nicht genügende physiologische Verträglichkeit aufwiesen oder nicht genügend hohe Löslichkeit oder Dispergierbarkeit in kosmetischen oder dermatologischen Zubereitungen aufwiesen oder auch sonstige Inkompatibilitäten mit kosmetischen oder dermatologischen Zubereitungen oder mehrere Nachteile zugleich.

Schaumförmige kosmetische Zubereitungen sind an sich bekannt. Schäume erlauben eine feine Verteilung von Wirkstoffen auf der Haut. Allerdings sind Schäume in der Regel nur durch Verwendung besonderer Tenside, welche darüberhinaus oft wenig hautverträglich sind, zu erreichen.

Nachschäumende Zubereitungen liegen oft in speziellen Ausführungsformen wie etwa nachschäumenden Rasiergelen oder dergleichen vor. Solche werden zunächst in fließförmiger Form aus einem Aerosolbehälter auf die Haut aufgetragen und entwickeln erst dort nach kurzer Verzögerung unter dem Einfluß des enthaltenen Nachschäummittels den eigentlichen Schaum. Der Vorteil solcher Zubereitungen gegenüber fertigen Schäumen beispielsweise,

welche bereits geschäumt aus dem Aerosolbehälter auf die Haut aufgebracht werden, liegt in einer besseren Benetzung.

Nachschäumende Zubereitungen sind also zwar an sich bekannt. Die US-PS 3,541,581 nennt als wesentliche Bestandteile einer solchen Zubereitung Wasser, Seife, (also wasserlösliche Salze höherer Fettsäuren), Gelstrukturbildner und Nachschäummittel. Auch andere Derartige Zubereitungen sind bekannt, die aber alle den Nachteil haben, kosmetisch unelegant zu sein und/oder die Anforderung an niedriges Reizpotential nicht zu erfüllen. Davon abgesehen waren speziell nachschäumende Lichtschutzzubereitungen dem Stande der Technik bisher gänzlich unbekannt, da der bekannte Stand der Technik im wesentlichen aus nachschäumenden Rasierzubereitungen und gegebenenfalls einigen Randprodukten zusammengesetzt wird.

Eine Aufgabe der vorliegenden Aufgabe war, den Stand der Technik in dieser Richtung zu bereichern.

Wenigstens einigen, wenn nicht allen diesen Nachteilen abzuhelfen, war also Aufgabe der vorliegenden Erfindung.

Es war indes überraschend und für den Fachmann nicht vorauszusehen, daß Wirkstoffkombinationen aus

(a) einer oder mehreren grenzflächenaktiven Substanzen, gewählt aus der Gruppe der Glucosederivate, welche sich durch die Strukturformel

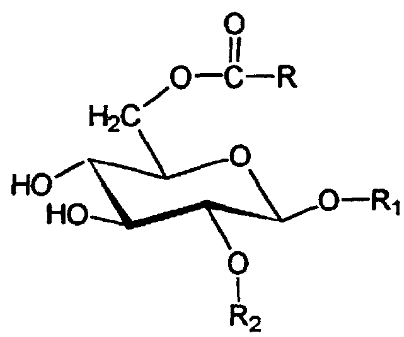

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) einer oder mehreren grenzflächenaktiven Substanzen, gewählt aus der Gruppe der Glycerin-mono- bzw. -dicarbonsäure-monoester der allgemeinen Formel

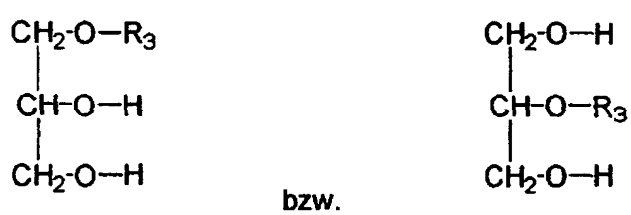

bzw.

wiedergegeben, wobei $R_3$ einen verzweigten oder unverzweigten Acylrest mit 6 - 24 Kohlenstoffatomen darstellt,

(c) einer oder mehreren wasserlöslicher, kosmetisch oder pharmazeutisch akzeptabler UV-Filtersubstanzen

den Nachteilen des Standes der Technik abhelfen.

Insbesondere wird die vorliegende Erfindung verkörpert durch kosmetische oder pharmazeutische Zubereitungen, welche Wirkstoffkombinationen aus

(a) einer oder mehreren grenzflächenaktiven Substanzen, gewählt aus der Gruppe der Glucosederivate, welche sich durch die Strukturformel

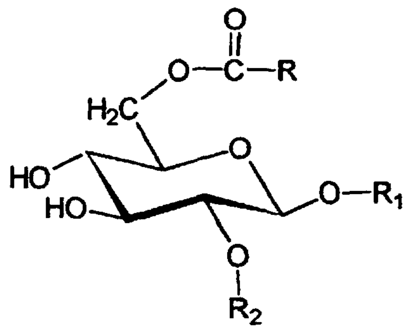

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) einer oder mehreren grenzflächenaktiven Substanzen, gewählt aus der Gruppe der Glycerin-mono- bzw. -dicarbonsäure-monoester der allgemeinen Formel

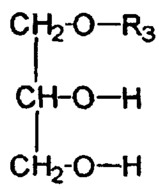

bzw.

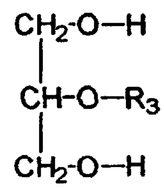

wiedergegeben, wobei $R_3$ einen verzweigten oder unverzweigten Acylrest mit 6 - 24 Kohlenstoffatomen darstellt,

(c) einer oder mehreren wasserlöslichen, kosmetisch oder pharmazeutisch akzeptabler UV-Filtersubstanzen,

(d) einer Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthält, und

(e) gegebenenfalls einer Ölphase, welche gegebenenfalls übliche, darin lösliche und/oder dispergierbare Substanzen, darunter insbesondere weitere kosmetisch oder pharmazeutisch akzeptable UV-Filtersubstanzen, enthält,

enthalten und in Form von Schäumen vorliegen.

Insbesondere stellt die Verwendung von Wirkstoffkombinationen aus

(a) einer oder mehrerer grenzflächenaktiven Substanzen, gewählt aus der Gruppe der Glucosederivate, welche sich durch die Strukturformel

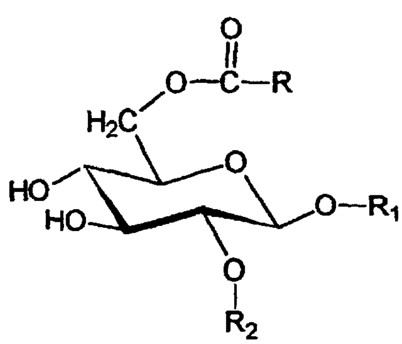

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Koh-

lenstoffatomen darstellt und wobei $R_2$ entweder ein Wasserstoffatom oder einen verzweigten oder unverzweigten Acylrest mit 1 bis 24 Kohlenstoffatomen darstellt.
(b) einer oder mehrerer grenzflächenaktiven Substanzen, gewählt aus der Gruppe der Glycerin-mono- bzw. -dicarbonsäure-monoester der allgemeinen Formel

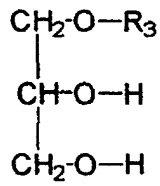

bzw.

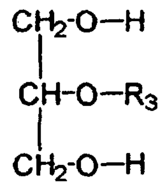

wiedergegeben, wobei $R_3$ einen verzweigten oder unverzweigten Acylrest mit 6 - 24 Kohlenstoffatomen darstellt,
(c) einer oder mehrerer wasserlöslicher, kosmetisch oder pharmazeutisch akzeptabler UV-Filtersubstanzen,
(d) einer Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthält, und
(e) gegebenenfalls einer Ölphase, welche gegebenenfalls übliche, darin lösliche und/oder dispergierbare Substanzen, darunter insbesondere weitere kosmetisch oder pharmazeutisch akzeptable UV-Filtersubstanzen, enthält,

zur Erzeugung kosmetischer oder pharmazeutischer Zubereitungen in Schaumform eine vorteilhafte Verkörperung der vorliegenden Erfindung dar.

Vorteilhaft wird R gewählt aus der Gruppe der unverzweigten Alkylreste, wobei der Myristylrest, der Palmitylrest, der Stearylrest und der Eicosylrest bevorzugt werden.

$R_1$ kann vorteilhaft ein Wasserstoffatom darstellen, wird aber bevorzugt aus der Gruppe Methyl-, Ethyl-, Propyl- und Isopropyl- gewählt.

$R_2$ kann vorteilhaft ein Wasserstoffatom darstellen, kann aber ebenfalls vorteilhaft aus der Gruppe Myristoyl-, Palmitoyl-, Stearoyl- und Eicosoyl- gewählt werden.

Besonders vorteilhaft wird als grenzflächenaktive Substanzen aus der Gruppe der Glucosederivate das Methylglucosesesquistearat gewählt, welches zu etwa gleichen Teilen aus den Substanzen

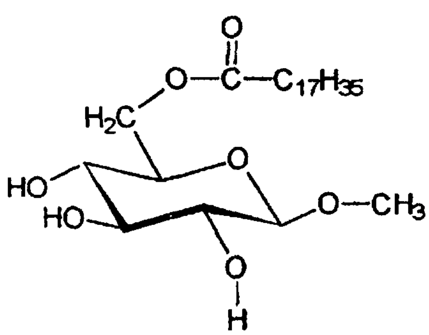

und

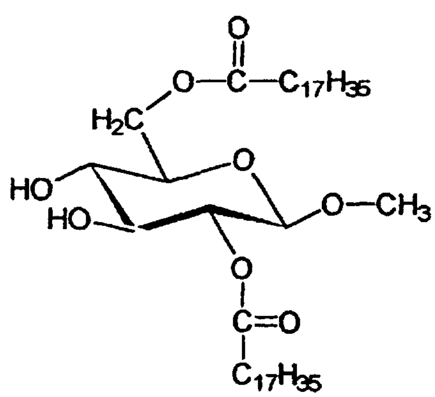

besteht. Solche Mischungen sind im Handel beispielsweise unter der Warenbezeichnung Tego® Care PS von der Gesellschaft Th.Goldschmidt KG erhältlich.

$R_3$ stellt bevorzugt einen Myristylrest, Palmitylrest, Stearylrest oder Eicosylrest dar.

Insbesondere vorteilhaft im Sinne der vorliegenden Erfindung hat sich als unter Punkt (b) aufgeführter grenzflächenaktiver Substanzen das Glycerylstearat herausgestellt, welches im Handel beispielsweise unter der Warenbezeichnung Tegin® M von der Gesellschaft Th.Goldschmidt KG erhältlich ist.

Vorteilhafte wasserlösliche UV-Filtersubstanzen im Sinne der vorliegenden Erfindung sind insbesondere solche, die an ihrem Molekülgerüst eine oder mehrere Sulfonsäuregruppen bzw. Sulfonatgruppen tragen. Bevorzugt sind insbesondere :

Die 2-Phenylbenzimidazol-5-sulfonsäure und ihre Salze, beispielsweise das Natrium-, Kalium- oder ihr Triethanolammonium-Salz

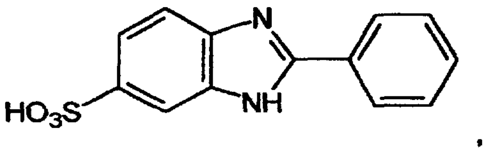

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

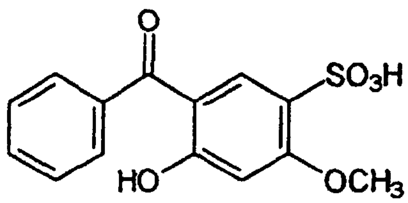

Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

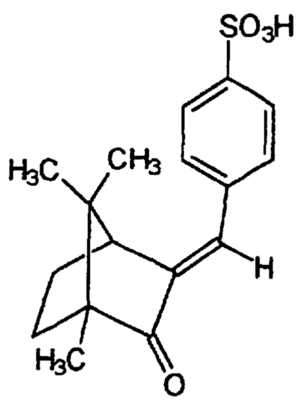

Die 2-Methyl-5-(2-oxo-3-bornylidenmethyl)benzolsulfonsäure und ihre Salze, beispielsweise das entsprechende Natrium-, Kalium- oder Triethanolammonium-Salz:

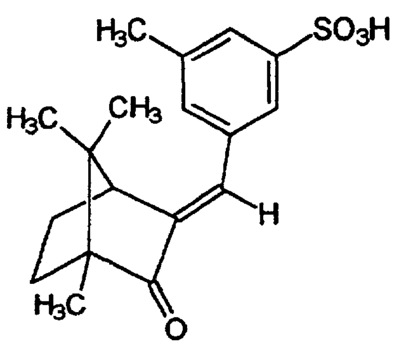

Das 1,4-di(2-oxo-10-Sulfo-3-bornylidenmethyl-)Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet:

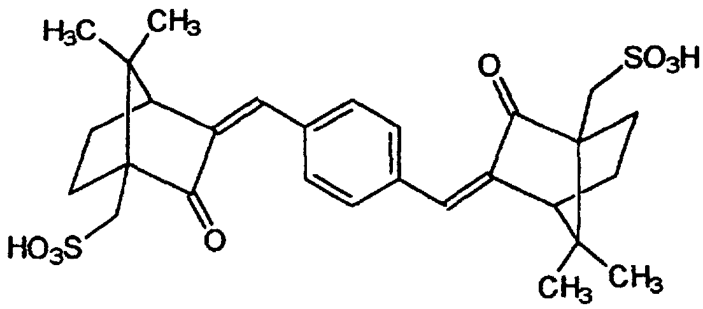

Die Phenylen-1,4-bis-(2-benzimidazyl)-3,3'-5,5'-tetrasulfonsäure:

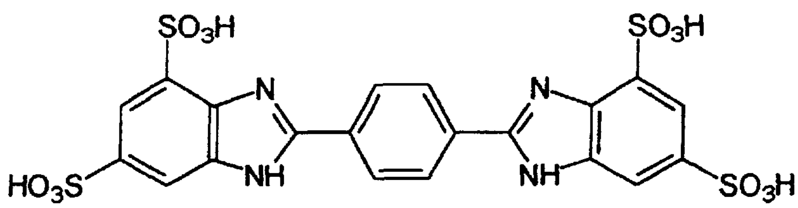

und ihre Salze, beispielsweise das entsprechende Natrium-, Kalium- oder Triethanolammonium-Salz insbesondere das Phenylen-1,4-bis-(2-benzimidazyl)-3,3'-5,5'-tetrasulfonsäure-bis-natriumsalz:

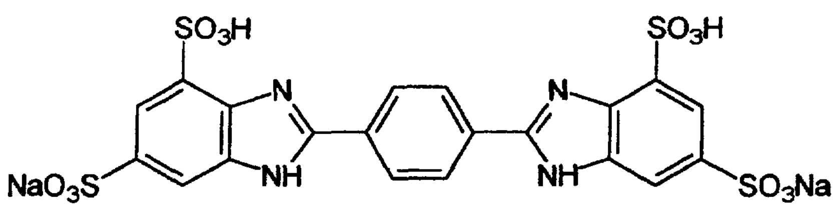

Erfindungsgemäße Zusammensetzungen stellen ungeschäumt, also unmittelbar nach Zusammengeben der Einzelkomponenten, Zweiphasensysteme, in der Regel Emulsionen, dar. Sie können bereits durch leichtes Verreiben, beispielsweise in den Händen oder beim Auftragen und Verreiben auf der Haut, aber auch durch Rühren oder sonstige Aufschäumvorgänge zu Schäumen gestaltet werden.

Erfindungsgemäß sind feinblasige, reiche Schäume von hervorragender kosmetischer Eleganz erhältlich. Bevorzugt stellen solche Schäume Lichtschutzzubereitungen dar, welche den geschilderten Nachteilen des Standes der Technik in überraschender Weise abhelfen. Erfindungsgemäß sind höhere Lichtschutzfaktoren erreichbar als der Stand der Technik dies hätte annehmen lassen.

In den erfindungsgemäßen kosmetischen oder dermatologischen Zubereitungen, haben ferner auch die schwererlöslichen Komponenten eine bessere Löslichkeit als in den Zubereitungen des Standes der Technik, auch dann, wenn mehrere solcher Komponenten vorliegen.

Erfindungsgemäß kann ferner die Agglomeration eventuell vorhandener anorganischer Pigmentpartikel (welche natürlich dispergiert, und nicht gelöst, vorliegen) mit den Folgen "Weißeln", Ausölen, Brechen der Emulsion verhindert werden, auch dann, wenn zusätzlich ein oder mehrere schwererlösliche Komponenten vorliegen.

Weiterhin sind erfindungsgemäß Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Insbesondere die Stabilität von 4-(tert.-Butyl)-4'-methoxydibenzoylmethan wird drastisch

erhöht.

Weiterhin sind erfindungsgemäß besonders gut hautverträgliche Zubereitungen erhältlich, wobei wertvolle Inhaltsstoffe besonders gut auf der Haut verteilt werden können.

Es ist erfindungsgemäß möglich die Einsatzmengen von 4,4',4"-(1,3,5-Triazin-2,4,6-triyltriimino)-tris-benzoesäure-tris(2-ethylhexylester), aber auch der anderen unter Normalbedingungen als Feststoff vorliegenden Lichschutzfiltersubstanzen, in kosmetischen oder dermatologischen Zubereitungen gegenüber dem Stande der Technik wesentlich zu erhöhen.

Andererseits erlaubt es die vorliegende Erfindung, bei geringerer Gesamtkonzentration an UV-Filtersubstanzen zu vergleichbarer oder sogar höherer Lichtschutzfilterwirkung zu gelangen, als es der Stand der Technik erlaubte. Dazu hat es sich als besonders vorteilhaft erwiesen, einen zusätzlichen Gehalt an kosmetischen bzw. pharmazeutisch akzeptablen Elektrolyten einzuführen.

Ferner war erstaunlich, daß erfindungsgemäß eine Stabilisierung von Lösungen des 4,4',4"-(1,3,5-Triazin-2,4,6-triyltriimino)-tris-benzoesäure-tris(2-ethylhexylesters) bewirkt wird, da die letztere Substanz nicht nur schlechte Löslichkeit aufweist, sondern auch aus ihrer Lösung leicht wieder auskristallisiert.

Die Gesamtmenge an der oder den wasserlöslichen UV-Filtersubstanzen in den fertigen kosmetischen oder dermatologischen Zubereitungen wird vorteilhaft aus dem Bereich von 0,1- 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure (sofern es diese Substanz ist, welche als wasserlösliche UV-Filtersubstanz im Sinne der vorliegenden Erfindung eingesetzt werden soll) bzw. deren Salze in den fertigen kosmetischen oder dermatologischen Zubereitungen wird, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäure (sofern es diese Substanz ist, welche als wasserlösliche UV-Filtersubstanz im Sinne der vorliegenden Erfindung eingesetzt werden soll) bzw. deren Salze in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure (sofern es diese Substanz ist, welche als wasserlösliche UV Filtersubstanz im Sinne der vorliegenden Erfindung eingesetzt werden soll) bzw. deren Salze in den fertigen kosmetischen oder dermatologischen Zubereitungen wird vorteilhaft aus dem Bereich von 0,1 - 15 Gew.-%, bevorzugt 0,5 - 10 Gew.-% gewählt, bezogen auf das Gesamtgewicht der Zubereitungen.

Die Gesamtmenge an 2-Methyl-5-(2-oxo-3-bornylidenmethyl)benzolsulfonsäure (sofern es diese Substanz ist, welche als wasserlösliche UV-Filtersubstanz im Sinne der vorliegenden Erfindung eingesetzt werden soll) bzw. deren Salze in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäure (sofern es diese Substanz ist, welche als wasserlösliche UV-Filtersubstanz im Sinne der vorliegenden Erfindung eingesetzt werden soll) bzw. deren Salze in den fertigen kosmetischen oder dermatologischen Zubereitungen wird vorteilhaft aus dem Bereich von 0,1 - 5,0 Gew.-%, bevorzugt 0,5 - 10,0 Gew.-% gewählt, bezogen auf das Gesamtgewicht der Zubereitungen.

Die Gesamtmenge an Phenylen-1,4-bis-(2-benzimidazyl)-3,3'-5,5'-tetrasulfonsäure (sofern es diese Substanz ist, welche als sulfonierte UV-Filtersubstanz im Sinne der vorliegenden Erfindung eingesetzt werden soll) bzw. deren Salzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4"-(1,3,5-Triazin-2,4,6-triyltriimino)-tris-benzoesäure-tris-(2-ethylhexylester) (als an sich fakultativ einzusetzender zusätzlicher UV-Filtersubstanz) in den fertigen kosmetischen oder dermatologischen Zubereitungen wird vorteilhaft aus dem Bereich von 0,1 - 10,0 Gew.-%, bevorzugt 0,5 - 6,0 Gew.-% gewährt, bezogen auf das Gesamtgewicht der Zubereitungen.

Die Gesamtmenge an 4-Methylbenzylidencampher (als an sich fakultativ einzusetzender zusätzlicher UV-Filtersubstanz) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat (als an sich fakultativ einzusetzender zusätzlicher UV-Filtersubstanz) in den fertigen kosmetischen oder dermatologischen Zubereitungen wird vorteilhaft aus dem Bereich von 0,1 - 15 Gew.-%. bevorzugt 0,5 - 7,5 Gew.-% gewählt, bezogen auf das Gesamtgewicht der Zubereitungen.

Die Gesamtmenge an Ethylhexyl-2-cyano-3,3-diphenylacrylat (als an sich fakultativ einzusetzender zusätzlicher UV-Filtersubstanz) in den fertigen kosmetischen oder dermatologischen Zubereitungen wird vorteilhaft aus dem Bereich von 0,1 - 15,0 Gew.-%, bevorzugt 0,5 - 10,0 Gew.-% gewählt, bezogen auf das Gesamtgewicht der Zubereitungen.

Die Gesamtmenge an 4-(tert.-Butyl)-4'-methoxydibenzoylmethan (als an sich fakultativ einzusetzender zusätzlicher UV-Filtersubstanz) in den fertigen kosmetischen oder dermatologischen Zubereitungen wird vorteilhaft aus dem Bereich von 0,1- 10,0 Gew.-%, bevorzugt 0,5 - 6,0 Gew.-% gewählt, bezogen auf das Gesamtgewicht der Zubereitungen.

Ferner ist vorteilhaft, die erfindungsgemäßen Wirkstoffkombinationen mit weiteren UVA- und/oder UVB-Filtern zu kombinieren.

Ferner kann gegebenenfalls von Vorteil sein, die erfindungsgemäßen Wirkstoffkombinationen mit weiteren UVA- und/oder UVB-Filtern zu kombinieren, beispielsweise bestimmten Salicylsäurederivaten wie

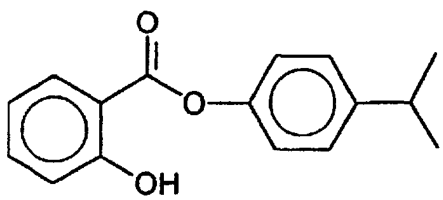

**(4-Isopropylbenzylsalicylat),**

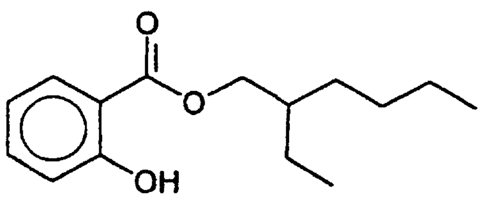

**(2-Ethylhexylsalicylat, Octylsalicylat),**

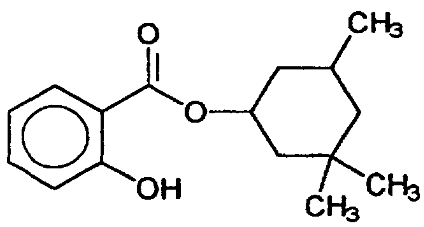

**(Homomenthylsalicylat).**

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Es kann auch von Vorteil sein, die erfindungsgemäßen Kombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Es ist erfindungsgemäß vorteilhaft, außer den erfindungsgemäßen Kombinationen weitere UVA-Filter und/oder UVB-Filter einzusetzen, wobei die Gesamtmenge der Filtersubstanzen z.B. 0,1 Gew.-% bis 30 Gew.-%, vorzugsweise 0,5 bis 10 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;

- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Die Liste der genannten UVB-Filter, die in Kombination mit den erfindungsgemäßen Wirkstoffkombinationen verwendet werden können, soll selbstverständlich nicht limitierend sein.

Erfindungsgemäße kosmetische und dermatologische Zubereitungen enthalten außerdem vorteilhaft, aber nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Die anorganischen Pigmente liegen bevorzugt in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa oder MT 100 T von der Firma Tayca oder M 160 von der Frima Kemira erhältlich.

Als wasserdispergierbare anorganische Mikropigmente könnnen beispielsweise solche Produkte gewählt werden, welche unter der Handelsbezeichnung Tioveil® von der Firma Tioxide erhältlich sind.

Die Gesamtmenge an anorganischen Pigmenten, insbesondere hydrophoben anorganischen Mikropigmenten (als an sich fakultativ einzusetzender zusätzlicher Substanz) in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Eine weitere vorteilhafte Ausführungsform der vorliegenden Erfindung besteht in After-Sun-Produkten, insbesondere als Pflegeschäume mit kühlender Wirkung.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Wie zuvor erwähnt, erlaubt es die vorliegende Erfindung, bei geringerer Gesamtkonzentration an UV-Filtersubstanzen zu vergleichbarer oder sogar höherer Lichtschutzfilterwirkung zu gelangen, als es der Stand der Technik bislang erlaubte. Dazu hat es sich als besonders vorteilhaft erwiesen, einen zusätzlichen Gehalt an kosmetische bzw. pharmazeutisch akzeptablen Elektrolyten einzuführen. Über weite Konzentrationsbereiche ist es möglich, die Konzentration der UV-Filtersubstanz oder -substanzen um den gleichen oder doch zumindest vergleichbaren Gehalt zu reduzieren mit dem die Rezeptur gleichsam mit einem oder mehreren Elektrolyten aufgefüllt wird. Als untere Grenze, bei dem sich dieses Verhalten in für den Anwender relevanter Weise bemerkbar macht, hat sich in der Regel ein Gesamtgehalt von etwa 0,5 Gew.-% an wasserlöslichen UV-Filtersubstanzen herausgestellt.

Die erfindungsgemäßen Zubereitungen enthalten daher bevorzugt Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Camosin, D-Camosin, L-Camosin und deren Derivate (z.B. Anserin), Carotinoide, Carotine (z.B. $\alpha$-Carotin, $\beta$ -Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprytylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, femer Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1** | |
|---|---|
| | Gew.-% |
| Glycerylstearat | 2,00 |
| Methylglucosesesquistearat | 5,00 |
| Caprylic/Capric Triglyceride | 5,00 |
| Octyldodecanol | 5,00 |
| Dicaprylylether | 2.00 |
| Phenylbenzimidazolsulfonsäure | 4,00 |
| Glycerin | 3,00 |
| Tocopherylacetat | 1,00 |
| NaOH | q.s. |
| Parfum, Konservierungsmittel | q.s. |
| Wasser | ad 100,00 |

| Beispiel 2 | |
|---|---|
| | Gew.-% |
| Glycerylstearat | 2,00 |
| Methylglucosesesquistearat | 5,00 |
| Caprylic/Capric Triglyceride | 1,67 |
| Octyldodecanol | 1,67 |
| Dicaprylylether | 1,67 |
| Phenylbenzimidazolsulfonsäure | 4,00 |
| Butylenglycol | 3,00 |
| Tocopherylacetat | 1,00 |
| NaOH | q.s. |
| Parfum, Konservierungsmittel | q.s. |
| Wasser | ad 100,00 |

| Beispiel 3 | |
|---|---|
| | Gew.-% |
| Treibmittel (Butan/Isobutan/Propan) | 5,00 |
| Glycerylstearat | 2,00 |
| Methylglucosesesquistearat | 5,00 |
| Caprylic/Capric Triglyceride | 1,67 |
| Octyldodecanol | 1,67 |
| Dicaprylylether | 1,67 |
| Phenylbenzimidazolsulfonsäure | 4,00 |
| Butylenglycol | 3,00 |
| Tocopherylacetat | 1,00 |
| NaOH | q.s. |
| Parfum, Konservierungsmittel | q.s. |
| Wasser | ad 100,00 |

| Beispiel 4 | |
|---|---|
| | Gew.-% |
| Glycerylstearat | 2,00 |
| Methylglucosesesquistearat | 5,00 |
| Caprylic/Capric Triglyceride | 5,00 |
| Octyldodecanol | 5,00 |
| Dicaprylylether | 1,67 |
| Benzol-1,4-di(2-oxo-3-bornylidenmethyl-10-Sulfonsäure) | 4,00 |
| Glycerin | 3,00 |
| Tocopherylacetat | 1,00 |
| NaOH | q.s. |
| Parfum, Konservierungsmittel | q.s. |
| Wasser | ad 100,00 |

| Beispiel 5 | |
|---|---|
| | Gew.-% |
| Glycerylstearat | 2,00 |

(fortgesetzt)

| Beispiel 5 | |
|---|---|
| | Gew.-% |
| Methylglucosesesquistearat | 5,00 |
| Caprylic/Capric Triglyceride | 1,67 |
| Octyldodecanol | 1,67 |
| $C_{12-15}$-Alkylbenzoate | 1,67 |
| Phenylbenzimidazolsulfonsäure | 2,00 |
| Tris-[anilino-(p-carbo-2'-ethyl-1'-hexyloxy)]-triazin | 3,00 |
| 4-(tert.-Butyl)-4'-methoxydibenzoylmethan | 2,00 |
| Butylenglycol | 3,00 |
| 4-Methyl-Benzylidencampher | 2,00 |
| Tocopherylacetat | 1,00 |
| NaOH | q.s. |
| Parfum, Konservierungsmittel | q.s. |
| Wasser | ad 100,00 |

**Patentansprüche**

**1.** Wirkstoffkombinationen aus

(a) einer oder mehreren grenzflächenaktiven Substanzen, gewählt aus der Gruppe der Glucosederivate, welche sich durch die Strukturformel

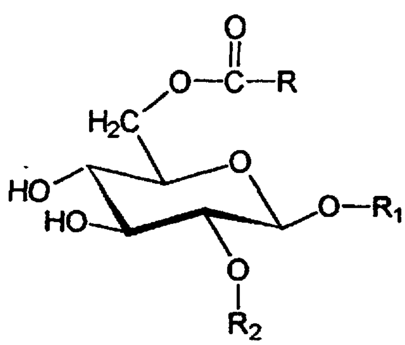

auszeichnen, wobei R einen verzweigten oder unverzweigten Alkylrest mit 1 bis 24 Kohlenstoffatomen darstellt, wobei $R_1$ entweder ein Wasserstoffatom oder einen verzweigten oder unverzweigten Alkylrest mit 1 bis 24 Kohlenstoffatomen darstellt und wobei $R_2$ entweder ein Wasserstoffatom oder einen verzweigten oder unverzweigten Acylrest mit 1 bis 24 Kohlenstoffatomen darstellt,

(b) einer oder mehreren grenzflächenaktiven Substanzen, gewählt aus der Gruppe der Glycerin-mono- bzw. -dicarbonsäure-monoester der allgemeinen Formel

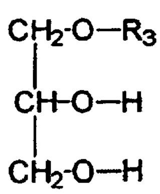

bzw.

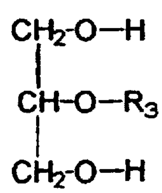

wiedergegeben, wobei $R_3$ einen verzweigten oder unverzweigten Acylrest mit 6 - 24 Kohlenstoffatomen darstellt,

(c) einer oder mehreren wasserlöslicher, kosmetisch oder pharmazeutisch akzeptabler UV-Filtersubstanzen.

**2.** Kosmetische oder pharmazeutische Zubereitungen, **gekennzeichnet dadurch, daß** sie in Form von Schäumen vorliegen und Wirkstoffkombinationen gemäß Anspruch 1 sowie

(d) eine Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthält, und
(e) gegebenenfalls eine Ölphase, welche gegebenenfalls übliche, darin lösliche und/oder dispergierbare Substanzen, darunter insbesondere weitere kosmetisch oder pharmazeutisch akzeptable UV-Filtersubstanzen, enthält,

enthalten.

**3.** Verwendung von Wirkstoffkombinationen gemäß Anspruch 1, unter Zusatz

(d) einer Wasserphase, welche gegebenenfalls übliche, darin lösliche und/oder dispergierbare Substanzen, darunter insbesondere weitere kosmetisch oder pharmazeutisch akzeptable UV-Filtersubstanzen, enthält, und
(e) gegebenenfalls einer Ölphase, welche gegebenenfalls übliche, darin lösliche und/oder dispergierbare Substanzen, darunter insbesondere weitere kosmetisch oder pharmazeutisch akzeptable UV-Filtersubstanzen, enthält,

zur Erzeugung kosmetischer oder pharmazeutischer Zubereitungen in Schaumform.

**4.** Wirkstoffkombinationen nach Anspruch 1, Zubereitungen nach Anspruch 2 oder Verwendung nach Anspruch 3, **dadurch gekennzeichnet, daß** in der Strukturformel des oder der grenzflächenaktiven Substanzen aus der Gruppe der Glucosederivate der Rest R gewählt wird aus der Gruppe Myristyl-, Palmityl-, Stearyl-, Eicosyl-, der Rest $R_1$ ein Wasserstoffatom darstellt oder aus der Gruppe Methyl-, Ethyl-, Propyl- und Isopropyl- gewählt wird, und/oder der Rest $R_2$ ein Wasserstoffatom darstellt oder aus der Gruppe Myristoyl-, Palmitoyl-, Stearoyl-, Eicosoyl-gewählt wird.

**5.** Wirkstoffkombinationen nach Anspruch 1, Zubereitungen nach Anspruch 2 oder Verwendung nach Anspruch 3, **dadurch gekennzeichnet, daß** als grenzflächenaktive Substanz aus der Gruppe der Glucosederivate das Methylglucosesesquistearat gewählt wird.

**6.** Wirkstoffkombinationen nach Anspruch 1, Zubereitungen nach Anspruch 2 oder Verwendung nach Anspruch 3, **dadurch gekennzeichnet, daß** als grenzflächenaktive Substanz aus der Gruppe der Glycerin-mono- bzw. -dicarbonsäure-monoester das Glycerylstearat gewählt wird.

**7.** Wirkstoffkombinationen nach Anspruch 1, Zubereitungen nach Anspruch 2 oder Verwendung nach Anspruch 3, **dadurch gekennzeichnet, daß** die wasserlösliche, kosmetisch oder pharmazeutisch akzeptabler UV-Filtersubstanz oder -substanzen gewählt werden aus der Gruppe der UV-Filtersubstanzen, die an ihrem Molekülgerüst eine oder mehrere Sulfonsäuregruppen bzw. Sulfonatgruppen tragen.

**8.** Wirkstoffkombinationen nach Anspruch 1, Zubereitungen nach Anspruch 2 oder Verwendung nach Anspruch 3, **dadurch gekennzeichnet, daß** die wasserlösliche, kosmetisch oder pharmazeutisch akzeptabler UV-Filtersubstanz oder -substanzen gewählt werden aus der Gruppe 2-Phenylbenzimidazol-5-sulfonsäure -sulfonsäure und ihre Salze, der Sulfonsäure-Derivate von Benzophenonen, vorzugsweise 2-Hydroxy-4-methoxybenzophenon-5-sulfonsäure und ihre Salze,der Sulfonsäure-Derivate des 3-Benzylidencamphers, wie z.B. 4-(2-Oxo-3-bomylidenmethyl)benzolsulfonsäure und ihre Salze, beispielsweise das entsprechende Natrium-, Kalium- oder Triethanolammonium-Salz 2-Methyl-5-(2-oxo-3-bornylidenmethyl)benzolsulfonsäure und ihre Salze, beispielsweise das entsprechende Natrium-, Kalium- oder Triethanolammonium-Salz, 1,4-di(2-oxo 1,4-di(2-oxo-10-Sulfo-3-bornylidenmethyl-)Benzol und dessen Salze.

## Claims

**1.** Combinations of agents, comprising:

a) one or more surface-active substances, chosen from the group of glucose derivates which exhibit the structural formula:

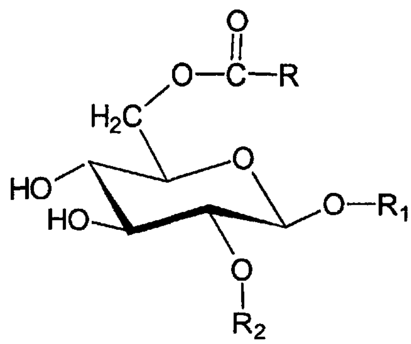

where R represents a branched or unbranched alkyl radical with from 1 to 24 carbon atoms, where $R_1$ represents either a hydrogen atom or a branched or unbranched alkyl radical with from 1 to 24 carbon atoms, and where $R_2$ represents either a hydrogen atom or a branched or unbranched acyl radical with from 1 to 24 carbon atoms.

b) one or more surface-active substances, chosen from the groups of glycerine mono-carbonic acid monoester or glycerine di-carbonic acid monoester exhibiting the generic formulae:

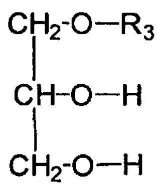

bzw.

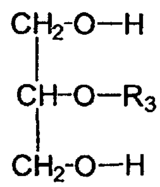

where $R_3$ represents a branched or unbranched acyl radical with from 6 to 24 carbon atoms.

c) one or more water-soluble, cosmetically or pharmaceutically acceptable UV filtering substances.

**2.** Cosmetic or pharmaceutical preparations, **characterised by** being in the form of a foam, and containing combinations of agents according to application 1, together with

d) a water-phase containing where necessary conventional dissolved and / or dispersed substances in particular including further cosmetically or pharmaceutically acceptable UV filtering substances, and

e) where necessary an oil phase containing where necessary conventional dissolved and / or dispersed substances in particular including further cosmetically or pharmaceutically acceptable UV filtering substances.

**3.** Use of combinations of agents according to application 1, with addition of

d) a water-phase containing where necessary conventional dissolved and / or dispersed substances in particular including further cosmetically or pharmaceutically acceptable UV filtering substances, and

e) where necessary an oil phase containing where necessary conventional dissolved and / or dispersed substances in particular including further cosmetically or pharmaceutically acceptable UV filtering substances

to produce cosmetic or pharmaceutical preparations in the form of foams.

**4.** Combinations of agents according to application 1, preparations according to application 2 or use according to application 3, **characterised by** the structural formula of surface active substances of the group of glucose derivates having R radicals, chosen from the groups myristyl, palmityl, stearyl, eicosyl, or having $R_1$ radicals exhibiting a hydrogen atom or chosen from the groups methyl, ethyl, propyl and isopropyl, and / or having $R_2$ radicals exhib-

iting a hydrogen atom or chosen from the groups myristyl, palmityl, stearyl, eicosyl.

**5.** Combinations of agents according to application 1, preparations according to application 2 or use according to application 3, **characterised by** methyl glucose sesquistearate being the surface active substance chosen from the group of glucose derivates.

**6.** Combinations of agents according to application 1, preparations according to application 2 or use according to application 3, **characterised by** glyceryl stearate being the surface active substance chosen from the group of glycerine mono-carbonic acid monoester or glycerine di-carbonic acid monoesters.

**7.** Combinations of agents according to application 1, preparations according to application 2 or use according to application 3, **characterised by** a water-soluble cosmetically or pharmaceutically acceptable UV filtering substance or substances chosen from the group of UV filtering substances that in their molecular make-up carry one or more sulphonic acid groups or suphonate groups.

**8.** Combinations of agents according to application 1, preparations according to application 2 or use according to application 3, **characterised by** a water-soluble cosmetically or pharmaceutically acceptable UV filtering substance or substances chosen from the group of 2-phenyl benzimidazol-5-sulphonic acid and its salts, the sulphonic acid derivatives of benzophenones, preferably 2-hydroxy-4-methoxy benzophenone-5-sulphonic acid and its salts, the sulphonic acid derivatives of benzylidene camphor, such as 4-(2-oxo-3-bornylidene methyl) benzol sulphonic acid and its salts, for instance the corresponding sodium, potassium, or triethyl ammonium salts, 2-methyl-5-(2-oxo-3-bornylidene methyl) benzol sulphonic acid and its salts, for instance the corresponding sodium, potassium, or triethyl ammonium salts, 1,4-di(2-oxo-10-sulpho-3-bornylidene methyl) benzol and its salts.

**Revendications**

**1.** Associations de substances actives au départ

(a) d'une ou plusieurs substances tensioactives, sélectionnées à partir de la classe des dérivés de glucose qui se caractérisent par la formule structurale suivante :

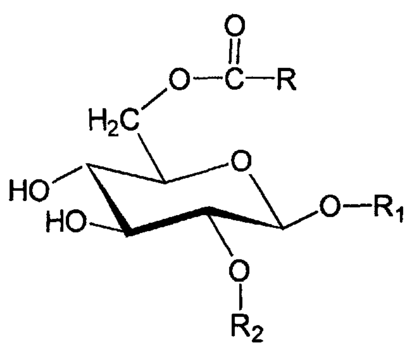

où R représente un radical alcoyle ramifié ou non ramifié ayant 1 à 24 atomes de carbone,
où $R_1$ représente soit un atome d'hydrogène soit un radical alcoyle ramifié ou non ramifié ayant 1 à 24 atomes de carbone,
et où $R_2$ représente soit un atome d'hydrogène soit un radical alcoyle ramifié ou non ramifié ayant 1 à 24 atomes de carbone ;
(b) d'une ou plusieurs substances tensioactives, sélectionnées à partir de la classe des monoesters mono- et dicarboxyliques de la glycérine représentés par la formule générale suivante :

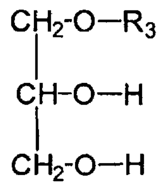

bzw.

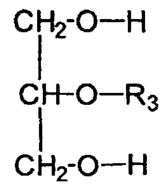

où R3 représente un radical alcoyle ramifié ou non ramifié ayant 6 à 24 atomes de carbone ;
(c) d'une ou plusieurs substances filtrantes d'UV solubles dans l'eau et acceptables aux plans cosmétique ou pharmaceutique.

**2.** Préparations cosmétiques ou pharmaceutiques **caractérisées par le fait qu'**elles sont présentes sous forme de mousses et qu'elles contiennent des associations de substances actives conformément à la revendication 1 ainsi que

(d) une phase aqueuse contenant le cas échéant des substances solubles et/ou dispersables dans cette phase, dont en particulier des substances filtrantes d'UV acceptables aux plans cosmétique ou pharmaceutique, et
(e) éventuellement une phase oléagineuse contenant le cas échéant des substances solubles et/ou dispersables dans cette phase, dont en particulier des substances filtrantes d'UV acceptables aux plans cosmétique ou pharmaceutique.

**3.** Utilisation d'associations de substances actives conformément à la revendication 1, avec en plus

(d) une phase aqueuse contenant le cas échéant des substances solubles et/ou dispersables dans cette phase, dont en particulier des substances filtrantes d'UV acceptables aux plans cosmétique ou pharmaceutique, et
(e) éventuellement une phase oléagineuse contenant le cas échéant des substances solubles et/ou dispersables dans cette phase, dont en particulier des substances filtrantes d'UV acceptables aux plans cosmétique ou pharmaceutique,

en vue de la production de préparations cosmétiques ou pharmaceutiques sous forme de mousse.

**4.** Associations de substances actives selon la revendication 1, préparations selon la revendication 2 ou utilisation selon la revendication 3, **caractérisées par le fait que** dans la formule structurale de la ou des substances tensioactives, l'on sélectionne le radical R dans la classe des dérivés du glucose, que dans la classe myristyle, palmityle, stearyle, eicosyle le radical $R_1$ représente un atome d'hydrogène ou que l'on sélectionne au sein du groupe méthyle, éthyle, propyle et isopropyle, et/ou que le radical $R_2$ représente un atome d'hydrogène ou que l'on sélectionne dans la classe myristoyle, palmitoyle, stearoyle, eicosoyle.

**5.** Associations de substances actives selon la revendication 1, préparations selon la revendication 2 ou utilisation selon la revendication 3, **caractérisées par le fait que** l'on sélectionne le méthyl glucose sesquistéarate dans la classe des dérivés du glucose en tant que substance tensioactive.

**6.** Associations de substances actives selon la revendication 1, préparations selon la revendication 2 ou utilisation selon la revendication 3, **caractérisées par le fait que** l'on sélectionne le glycéryl stéarate dans la classe des monoesters mono- et dicarboxyliques de la glycérine en tant que substance tensioactive.

**7.** Associations de substances actives selon la revendication 1, préparations selon la revendication 2 ou utilisation selon la revendication 3, **caractérisées par le fait que** l'on sélectionne une ou plusieurs substances filtrantes d'UV solubles dans l'eau et acceptables aux plans cosmétique ou pharmaceutique dans la classe des substances filtrantes d'UV qui présentent dans leur charpente moléculaire une ou plusieurs classes d'acides sulfoniques et classes de sulfonates.

**8.** Associations de substances actives selon la revendication 1, préparations selon la revendication 2 ou utilisation

selon la revendication 3, **caractérisées par le fait que** l'on sélectionne une ou plusieurs substances filtrantes d'UV solubles dans l'eau et acceptables aux plans cosmétique ou pharmaceutique dans la classe de l'acide 2-phénylbenzimidazole-5-sulfonique et ses sels, des dérivés de l'acide sulfonique de benzophénones, de préférence l'acide 2-hydroxy-4-méthoxybenzophénone-5-sulfonique et ses sels, des dérivés de l'acide sulfonique du 3-benzylidène camphre comme par exemple l'acide 4-(2-oxo-3-bornylidèneméthyl)-benzènesulfonique et ses sels, par exemple le sel correspondant de potassium, de sodium et de triéthanolamine, l'acide 2-méthyl5-(2-oxo-3-bornylidèneméthyl)-benzènesulfonique et ses sels, par exemple le sel correspondant de potassium, de sodium et de triéthanolamine, et l'acide 1,4-di(2-oxo-10-sulfo-3- bornylidèneméthyl)-benzène et ses sels.